Europäisches Patentamt

(19) European Patent Office

Office européen des brevets

(11) Numéro de publication: **0 231 689**
**B1**

(12) **FASCICULE DE BREVET EUROPEEN**

(45) Date de publication du fascicule du brevet:
03.10.90

(51) Int. Cl.⁵: **C07C 57/04**, C07C 51/567

(21) Numéro de dépôt: **86402759.4**

(22) Date de dépôt: **11.12.86**

(54) **Procédé de synthèse d'anhydrides (méth)acryliques.**

(30) Priorité: **24.12.85 FR 8519116**

(43) Date de publication de la demande:
**12.08.87 Bulletin 87/33**

(45) Mention de la délivrance du brevet:
**03.10.90 Bulletin 90/40**

(84) Etats contractants désignés:
**AT BE CH DE ES GB GR IT LI LU NL SE**

(56) Documents cités:
**DE-B- 1 102 142**
**FR-A- 2 246 538**
**US-A- 3 960 900**
**US-A- 4 029 592**

(73) Titulaire: **NORSOLOR S.A., Tour Aurore Place des Reflets, F-92080 Paris la Défense Cédex 5(FR)**

(72) Inventeur: **Hurtel, Patrice, 2 chemin des Brassens Résidence Foch, F-57600 Saint Avold(FR)**
Inventeur: **Laurent, Denis, 31 Parc du Tyrol, F-57500 Saint Avold(FR)**
Inventeur: **Rondini, Joseph, 82 rue de Guerting, F-57880 Ham Sous Varsberg(FR)**

(74) Mandataire: **Rieux, Michel, c/o NORSOLOR Service Propriété Industrielle B.P. 57, F-62670 Mazingarbe(FR)**

## Description

La présente invention concerne un nouveau procédé de synthèse d'anhydrides (méth)acryliques. Par anhydrides (méth)acryliques, on entend l'anhydride méthacrylique ou l'anhydride acrylique.

On connaît actuellement, notamment par le brevet FR-A-2 246 538, un procédé de synthèse d'anhydrides (méth)acryliques selon lequel on fait réagir l'acide (méth)acrylique sur l'anhydride acétique en présence d'un catalyseur, par exemple un acide fort comme l'acide sulfurique, ou encore du chlorure de zinc. Le principal problème posé par ce procédé, mais d'importance, réside dans la formation d'une impureté qui favorise la polymérisation du milieu réactionnel. Des analyses ont montré que cette impureté est principalement composée de dimère cyclique d'acide (méth)acrylique. Allant plus avant dans ces recherches, la demanderesse a mis au point un nouveau procédé de synthèse d'anhydrides (méth)acryliques selon lequel, de façon surprenante, en l'absence de catalyseur on obtient d'excellents rendements en anhydrides (méth)acryliques avec une réduction considérable de la formation de l'impureté gênante.

Plus précisément, le procédé objet de la présente invention consiste à faire réagir de l'acide (méth)acrylique sur de l'anhydride acétique en l'absence de catalyseur et en présence d'un inhibiteur de polymérisation.

L'anhydride (méth)acrylique formé est ensuite de préférence séparé par distillation.

Conformément à un mode de réalisation du procédé selon l'invention, l'acide acétique formé est soutiré pendant la réaction.

La température de réaction est maintenue entre 60 et 170°C, et de préférence, entre 80 et 110°C.

La pression lors de la réaction, est maintenue entre la pression atmosphérique et 0,013 bar, et de préférence, entre 0,39 et 0,065 bar.

Conformément à un mode de réalisation préféré du procédé selon l'invention, dans le but de faciliter le soutirage de l'acide acétique et donc la formation d'anhydrides (méth)acryliques, on maintient la température de réaction constante et on diminue la pression d'une valeur d'écart comprise généralement entre 0,013 et 0,13 bar pendant la réaction, ou, d'une manière différente, on maintient la pression constante et on augmente la température de réaction d'une valeur d'écart comprise généralement entre 5 et 50°C.

Le rapport molaire entre l'acide (méth)acrylique et l'anhydride acétique est choisi entre 0,5 et 5, et de préférence entre 2 et 2,2.

Les acides (méth)acryliques concernés par la présente invention sont l'acide acrylique et l'acide méthacrylique.

Parmi les inhibiteurs de polymérisation, on peut utiliser la phénothiazine, l'hydroquinone, le bleu de méthylène, le sulfate de fer, un sel de cuivre tel que l'acétate de cuivre, le sulfate de cuivre dans une quantité supérieure ou égale à 1500 ppm.

Le procédé selon l'invention permet d'atteindre des rendements élevés et le plus souvent supérieurs à 80%.

Les anhydrides (méth)acryliques servent notamment de réactifs dans la synthèse de (méth)acrylamide comme le diméthylaminopropyl(méth)acrylamide ou encore dans la synthèse de (méth)acrylates, par exemple les (méth)acrylates fluorés comme le (méth)acrylate de trifluoroéthyle.

Les exemples donnés ci-dessous à titre indicatif permettront de mieux comprendre l'invention.

EXAMPLE 1 : Synthèse de l'anhydride méthacrylique

Dans un réacteur muni d'un système d'agitation mécanique, chauffé électriquement et surmonté d'une colonne à distiller, on introduit la charge suivante (en parties en poids) :
- acide méthacrylique : 430
- anhydride acétique : 255
- phénothiazine : 2000 ppm
- bleu de méthylène : 2000 ppm

On porte le mélange réactionnel sous agitation et tout au long de la réaction qui dure 3 h 15 on soutire l'acide acétique entre 48 - 63°C, la pression étant de 0,13 bar au début de la réaction, et de 0,065 bar en fin de réaction. Après réaction, on effectue la distillation. On distille en tête l'acide méthacrylique et l'anhydride acétique n'ayant pas réagi de 50 à 107°C sous 0,045 bar.

Cette fraction représente 70 parties (en poids). Ensuite, on distille l'anhydride méthacrylique à 108°C sous 0,045 bar. On recueille 335 parties (en poids) d'anhydride méthacrylique.

Le rendement est de 87%.

EXEMPLE 2 : Synthèse de l'anhydride acrylique

Dans un montage identique à celui décrit dans l'Exemple 1, on introduit la charge suivante (en parties en poids) :
- acide acrylique : 432
- anhydride acétique : 306
- bleu de méthylène : 2000 ppm
- phénothiazine : 2000 ppm

On porte le mélange réactionnel sous agitation et, tout au long de la réaction qui dure 4 heures, on soutire l'acide acétique sous une pression de 0,13 bar, la température étant de 85°C au début de la réaction et de 105°C en fin de réaction. Après réaction, on effectue la distillation. On distille en tête notamment l'anhydride acétique et l'acide acrylique n'ayant pas réagi sous 0,039 bar à une température que l'on fait varier entre 75 à 120°C. Cette fraction représente 75 parties (en poids).

Ensuite, on distille l'anhydride acrylique à 80°C sous 0,032 bar. On recueille 302 parties (en poids) d'anhydride acrylique.

Le rendement est de 80%.

## Revendications

1. Procédé de synthèse d'anhydride (méth)acrylique par réaction de l'acide (méth)acrylique sur l'anhydride acétique caractérisé en ce que ladite réaction est effectuée en l'absence de catalyseur et en présence d'un inhibiteur de polymérisation, et en ce que le rapport molaire entre l'acide (méth)-acrylique et l'anhydride acétique est compris entre 0,5 et 5.

2. Procédé selon la revendication 1, caractérisé en ce que pendant la réaction, on soutire l'acide acétique formé.

3. Procédé selon la revendication 1 ou 2, caractérisé en ce que la température de réaction est maintenue entre 60 et 170°C.

4. Procédé selon la revendication 3, caractérisé en ce que la température de réaction est maintenue entre 80 et 110°C.

5. Procédé selon la revendication 1 ou 2, caractérisé en ce que la pression lors de la réaction est maintenue entre la pression atmosphérique et 0,013 bar.

6. Procédé selon la revendication 5, caractérisé en ce que la pression est maintenue entre 0,39 et 0,065 bar.

7. Procédé selon l'une quelconque des revendications 1 à 6, caractérisé en ce que, pendant la réaction, on maintient la température constante et on diminue la pression d'une valeur d'écart comprise entre 0,013 et 0,13 bar.

8. Procédé selon l'une quelconque des revendications 1 à 6, caractérisé en ce que, pendant la réaction, on maintient la pression constante et on augmente la température d'une valeur d'écart comprise entre 5 et 50°C.

9. Procédé selon l'une quelconque des revendications 1 à 8, caractérisé en ce que le rapport molaire entre l'acide (méth)acrylique et l'anhydride acétique est compris entre 2 et 2,2.

10. Procédé selon l'une quelconque des revendications 1 à 9, caractérisé en ce que la quantité d'inhibiteur de polymérisation est supérieure ou égale à 1500 ppm.

## Claims

1. Process for the synthesis of (meth)acrylic anhydride by the reaction of (meth)acrylic acid with acetic anhydride, characterized in that the said reaction is performed in the absence of catalyst and in the presence of a polymerization inhibitor, and in that the mole ratio of (meth)acrylic acid to acetic anhydride is between 0.5 and 5.

2. Process according to Claim 1, characterized in that, during the reaction, the acetic acid formed is drawn off.

3. Process according to Claim 1 or 2, characterized in that the reaction temperature is maintained at between 60 and 170°C.

4. Process according to Claim 3, characterized in that the reaction temperature is maintained at between 80 and 110°C.

5. Process according to Claim 1 or 2, characterized in that the pressure during the reaction is maintained at between atmospheric pressure and 0.013 bar.

6. Process according to Claim 5, characterized in that the pressure is maintained at between 0.39 and 0.065 bar.

7. Process according to any one of Claims 1 to 6, characterized in that, during the reaction, the temperature is maintained constant and the pressure is decreased by a difference value of between 0.013 and 0.13 bar.

8. Process according to any one of Claims 1 to 6, characterized in that, during the reaction, the pressure is maintained constant and the temperature is increased by a difference value of between 5 and 50°C.

9. Process according to any one of Claims 1 to 8, characterized in that the mole ratio of (meth)acrylic acid to acetic anhydride is between 2 and 2.2.

10. Process according to any one of Claims 1 to 9, characterized in that the quantity of polymerization inhibitor is greater than or equal to 1,500 ppm.

## Patentansprüche

1. Verfahren zur Synthese von (Meth)acrylsäureanhydrid durch Umsetzung von (Meth)acrylsäure mit Essigsäureanhydrid, dadurch gekennzeichnet, daß man die Umsetzung in Abwesenheit eines Katalysators und in Gegenwart eines Polymerisations-inhibitors durchführt, und daß das Molverhältnis zwischen der (Meth)acrylsäure und dem Essigsäureanhydrid zwischen 0,5 und 5 beträgt.

2. Verfahren nach Anspruch 1, dadurch gekennzeichnet, daß man während der Reaktion die gebildete Essigsäure abzieht.

3. Verfahren nach Anspruch 1 oder 2, dadurch gekennzeichnet, daß man die Reaktionstemperatur zwischen 60° und 170°C hält.

4. Verfahren nach Anspruch 3, dadurch gekennzeichnet, daß man die Reaktionstemperatur zwischen 80° und 110°C hält.

5. Verfahren nach Anspruch 1 oder 2, dadurch gekennzeichnet, daß man den Druck während der Reaktion zwischen Atmosphärendruck und 0,013 bar hält.

6. Verfahren nach Anspruch 5, dadurch gekennzeichnet, daß man den Druck zwischen 0,39 und 0,065 bar hält.

7. Verfahren nach einem der Ansprüche 1-6, dadurch gekennzeichnet, daß man während der Reaktion die Temperatur konstant hält und den Druck um einen Abweichungswert zwischen 0,013 und 0,13 bar verringert.

8. Verfahren nach einem der Ansprüche 1-6, dadurch gekennzeichnet, daß man während der Reaktion den Druck konstant hält und die Temperatur um einen Abweichungswert zwischen 5° und 50°C erhöht.

9. Verfahren nach einem der Ansprüche 1-8, dadurch gekennzeichnet, daß das Molverhältnis zwischen der (Meth)acrylsäure und dem Essigsäureanhydrid zwischen 2 und 2,2 beträgt.

10. Verfahren nach einem der Ansprüche 1-9, dadurch gekennzeichnet, daß die Menge des Polymerisations-inhibitors größer als oder gleich 1500 TpMill. beträgt.